# EUROPÄISCHE PATENTSCHRIFT

(11) **EP 0 901 501 B1**
(45) Veröffentlichungstag und Bekanntmachung des Hinweises auf die Patenterteilung: **17.10.2001**
(21) Anmeldenummer: 97922946.5
(22) Anmeldetag: 02.05.1997
(51) Int. Cl.: C07H 21/00, C07H 19/04

(54) **NUCLEOSID-DERIVATE MIT PHOTOLABILEN SCHUTZGRUPPEN**
NUCLEOSIDE DERIVATIVES WITH PHOTO-UNSTABLE PROTECTIVE GROUPS
DERIVES DE NUCLEOSIDES A GROUPES PROTECTEURS PHOTOLABILES

(30) Priorität: 20.05.1996 DE 19620170
(43) Veröffentlichungstag der Anmeldung: 17.03.1999
(73) Patentinhaber: Pfleiderer, Wolfgang, 78464 Konstanz (DE)
(72) Erfinder: PFLEIDERER, Wolfgang, D-78464 Konstanz (DE); Bühler, Sigrid, D-78464 Konstanz (DE)
(74) Vertreter: HOFFMANN - EITLE
(86) Internationale Anmeldenummer: EP9702257
(87) Internationale Veröffentlichungsnummer: WO9744345

(56) Entgegenhaltungen:
- WO-A-96/18634
- DE-A- 3 606 394
- DE-A- 3 606 395
- H.SCHIRMEISTER ET AL.: "21.Nucleosides" HELVETICA CHIMICA ACTA., Bd. 76, Nr. 1, 1993, BASEL CH, Seiten 385-401, XP002041322
- E.UHLMANN ET AL.: "New Improvements in Oligonucleotide Synthesis by Use of the P-nitrophenylethyl Phosphate Blocking Group and its Deprotection by DBU or DBN." TETRAHEDRON LETTERS., Bd. 21, Nr. 13, 1980, OXFORD GB, Seiten 1181-1184, XP002041323
- PFLEIDERER W ET AL: "NEW PROTECTING GROUPS IN NUCLEOSIDE AND NUCLEOTIDE CHEMISTRY" BIOPHOSPHATES AND THEIR ANALOGUES - SYNTHESIS, STRUCTURE, METABOLISM AND ACTIVITY, 1.Januar 1987, Seiten 133-142, XP000570661 in der Anmeldung erwähnt

## Beschreibung

Gegenstand der vorliegenden Erfindung sind Nucleosid-Derivate mit photolabilen Schutzgruppen und Verfahren zu deren Herstellung.

Photolabile Schutzgruppen für die Hydroxy- und Phosphatfunktionen in Nucleosiden bzw. Nucleotiden sind von besonderem Interesse, da sie bspw. für lichtgesteuerte Parallel-Synthesen von Oligonucleotiden auf einem festen Träger geeignet sind (vgl. S.P.A. Fodor et al. Science 1991, 251, S. 767 ff). Mit ihrer Hilfe können sogenannte DNA-Chips (d. h. Trägerplättchen, auf deren Oberfläche viele verschiedene Oligonucleotide angeordnet sind) hergestellt werden, die wiederum in der Molekularbiologie für eine schnelle DNA-Sequenz-Analyse benötigt werden.

Entsprechend dem Stand der Technik wurden bisher als photolabile Schutzgruppen in der Nucleosid- bzw. Nucleotidchemie vor allem die o-Nitrobenzyl-Gruppe und ihre Derivate eingesetzt (vgl. V.N.R. Pillai, Org. Photochem. 1987, 9, S. 225 ff bzw. J.W. Walker et al., J. Am. Chem. Soc. 1988, 110, S. 7170 ff). Als besonders nachteilig bei diesen Schutzgruppen hat sich die langsame und zum Teil nur unvollständige Entschützung der entsprechenden Nucleosid- bzw. Nucleotid-Derivate erwiesen. Außerdem entstehen bei der Abspaltung der o-Nitrobenzyl-Verbindungen z. T. unerwünschte Nebenprodukte in Form von toxischen Nitrosophenylverbindungen.

Als weitere photolabile Schutzgruppe für Nucleoside wurde entsprechend dem Artikel von W. Pfleiderer et al. in "Biophosphates and Their Analogues-Synthesis, Structure, Metabolism and Activity", Elsevier Science Publishers B.V. (Amsterdam) 1987, S. 133ff die 2-(o-Nitrophenyl)ethylgruppe empfohlen, die jedoch ausschließlich als Schutzgruppe im Basenteil, insbesondere in O⁶-Stellung des Guanosins, eingeführt wird. In derselben Publikation werden auch die p-Nitrophenylethoxycarbonyl (NPEOC)- und die 2,4-Dinitrophenylethoxycarbonyl (DNPEOC)-Gruppen sowohl als Schutzgruppen für die Aminofunktionen als auch für die Hydroxylfunktionen im Zuckerteil beschrieben, doch erfolgt die Abspaltung dieser Gruppen ausschließlich durch basenkatalysierte β-Eliminierung.

Der vorliegenden Erfindung lag daher die Aufgabe zugrunde, Nucleosid-Derivate mit photolabilen Schutzgruppen für die 5'-OH-Funktion im Zuckerteil zu entwickeln, welche die genannten Nachteile entsprechend dem Stand der Technik nicht aufweisen, sondern sich vergleichsweise schnell, quantitativ und ohne Bildung unerwünschter Nebenprodukte entschützen lassen.

Diese Aufgabe wurde erfindungsgemäß durch Nucleosid-Derivate der allgemeinen Formel (I) entsprechend Anspruch 1 gelöst. Es hat sich nämlich überraschenderweise gezeigt, daß sich die erfindungsgemäßen Schutzgruppen wesentlich schneller und vollständiger abspalten lassen als z. B. die o-Nitrobenzylgruppen. Außerdem konnten bei der Entschützung bisher keine Nebenprodukte in größerem Umfang festgestellt werden, was ebenfalls nicht vorhersehbar war.

Die erfindungsgemäßen Nucleosid-Derivate weisen folgende allgemeine Formel (I) auf: 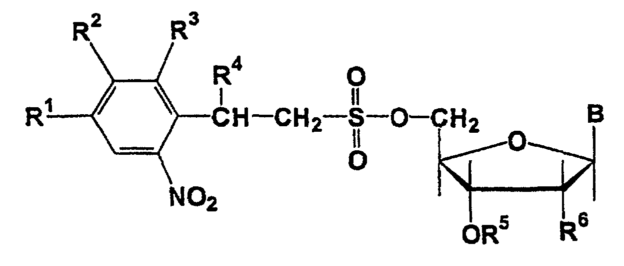 wobei die Reste R¹, R² und R³ am Phenylring folgende Bedeutung haben können:
- R¹ =: H, NO₂, CN, OCH₃, Halogen oder Alkyl oder Alkoxyalkyl mit 1 bis 4 C-Atomen
- R² =: H, OCH₃
- R³ =: H, F, Cl, Br, NO₂ oder ein aliphatischer Acylrest mit 2 bis 5 C-Atomen (wie z. B. Acetyl)

Der Rest R⁴, der am C₂-Atom der o-Nitrophenylethyl-Gruppierung sitzt, kann entweder H, Halogen, OCH₃, ein Alkylrest mit 1 bis 4 C-Atomen oder ein ggf. substituierter Arylrest sein. Der Alkylrest kann hierbei linear oder verzweigt, substituiert (insbesondere mit einem oder mehreren Halogenatomen) oder unsubstituiert sowie gesättigt oder ungesättigt sein; das gleiche gilt auch für die Alkyl- und Alkoxyalkylreste bei R¹. Vorzugsweise stellt R⁴ einen Methylrest dar. Der Arylrest stellt vorzugsweise eine Phenylgruppe dar, die ggf. noch mit Alkyl- (mit 1 bis 4 C-Atomen), Alkoxy-(z. B. Methoxy) oder Dialkylamino-Gruppen (z. B. Dimethylamino) bzw. F, Cl, Br, NO₂ oder CN substituiert sein kann. Im Falle von R⁴ ≠ H sind die Substituenten R¹, R² und R³ am Phenylring vorzugsweise Wasserstoffreste.

Halogen bedeutet in dieser Anmeldung durchwegs F, Cl, Br, I und vorzugsweise F, Cl oder Br.

Der Nucleosidteil der erfindungsgemäßen Verbindungen besteht aus den üblichen D-Ribofuranose- bzw. 2'-Desoxyribofuranose-Einheiten sowie den Pyrimidin- (B = Cytosin, Thymin, Uracil) bzw. Purinbasen (B = Adenin, Guanin). Als Basen können auch 2,6-Diaminopurin-9-yl-, Hypoxanthin-9-yl-, 5-Methylcytosin-1-yl-, 5-Amino-4-imidazolcarbonsäureamid-1-yl- oder 5-Amino-4-imidazolcarbonsäureamid-3-yl-Reste eingesetzt werden.

Die OH-Gruppe(n) im Ribofuranosid- bzw. 2'-Desoxyribofuranose-Teil können je nach Bedarf frei oder geschützt sein. Zum Schutz der 3'-Stellung haben sich hierbei die bekannten Phosphitamid-Gruppen bewährt, wie z. B. 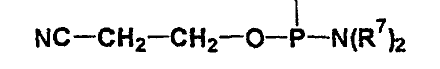 oder 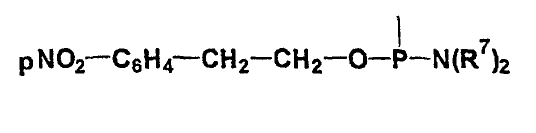 wobei die R⁷-Gruppen gleich oder verschieden sein können und lineare oder verzweigte Alkylreste mit 1 bis 4 C-Atomen bedeuten. Vorzugsweise sind sie Ethyl- oder Isopropylreste.

In der 2'-Stellung des Ribofuranosid-Teiles (Position R⁶) kann neben dem Wasserstoff- oder Halogenatom (insbesondere F, Cl, Br) eine freie oder geschützte OH-Gruppe vorliegen, wobei eine beliebige in der Nucleotidchemie übliche Schutzgruppe (R⁸) verwendet werden kann. Insbesondere kann auf die üblichen Alkyl-, Alkenyl-, Acetal- oder Silylether-Schutzgruppen für Sauerstoffatome (X = O) zurückgegriffen werden. R⁶ kann auch eine S-Alkylgruppe darstellen (X = S, R⁸ = Alkyl). Bevorzugte Beispiele für O-Alkyl-Schutzgruppen sind O-Methyl- oder O-Ethylreste, für O-Alkenyl-Schutzgruppen O-Allylreste, für O-Acetal-Schutzgruppen O-Tetrahydropyranyl- bzw. O-Methoxytetrahydropyranyl-Reste sowie für O-Silylether-Schutzgruppen O-t-Butyldimethylsilyl-Reste.

Gemäß einer bevorzugten Ausführungsform können die Pyrimidin- bzw. Purinbasen mit primären Aminofunktionen (z. B. Adenin, Cytosin und Guanin) noch permanente Schutzgruppen vorzugsweise auf Carbonylbasis aufweisen). Bevorzugt sind hierbei vor allem Phenoxyacetyl- oder Dimethylformamidino-Reste, die für alle drei genannten Basen in Frage kommen. Daneben gibt es noch spezielle Schutzgruppen, die nur bei bestimmten Basen eingeführt werden. Dies sind bspw. im Falle von Adenin Benzoyl- oder p-Nitrophenylethoxycarbonyl (p-NPEOC)-Reste. Für Guanin können neben den p-NPEOC-Resten auch Isobutyroyl- oder p-Nitrophenylethyl- (p-NPE)-Schutzgruppen eingeführt werden. Schließlich eignen sich für Cytosin neben den p-NPEOC-Resten auch noch Benzoyl-Schutzgruppen.

Die Herstellung der erfindungsgemäßen Nucleosid-Derivate kann in drei Stufen erfolgen. In der ersten Stufe a) wird ein Alkohol der allgemeinen Formel (II) 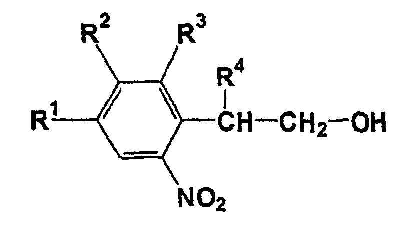 in der R¹, R², R³, R⁴ die oben angegebene Bedeutung besitzen, mit Thionylchlorid vorzugsweise in einem unpolaren organischen Lösemittel bei Temperaturen zwischen 50 und 120 °C ggf. in Anwesenheit einer Base zur Umsetzung gebracht.

Die Alkoholkomponente ist in den meisten Fällen bekannt oder kann in analoger Weise nach bekannten Verfahren hergestellt werden. Als unpolares organisches Lösemittel wird in Stufe a) vorzugsweise Toluol und als Base vorzugsweise Pyridin in einer Menge von 2 bis 10 Vol.-% bezogen auf das eingesetzte Toluol verwendet. Die Reaktionskomponenten können zwar in annähernd stöchiometrischem Verhältnis zur Umsetzung gebracht werden, doch wird das Thionylchlorid vorzugsweise in deutlichem Überschuß, bspw. in zwei- bis fünffachern molaren Überschuß, bezogen auf die Alkoholkomponente eingesetzt. Auch die Konzentration der Alkoholkomponente kann in weiten Grenzen variiert werden, doch hat es sich als besonders vorteilhaft erwiesen, diese Konzentration auf 1,0 bis 20,0 mmol pro 10 ml Solvens einzustellen.

Bei dieser Reaktion (Reaktionsdauer ca. 1 bis 3 Std.) entstehen in guter Reinheit und hoher Ausbeute (> 85 %) die entsprechenden Phenylalkylchloride der allgemeinen Formel (III)

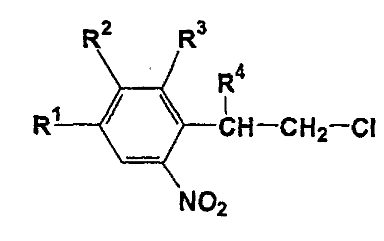

Die Aufarbeitung der entsprechenden Produkte erfolgt vorzugsweise, indem man zunächst die Reaktionslösung mit Eiswasser und ggf. mehrmals mit Chloroform oder Dichlormethan behandelt, die organischen Phasen (bspw. mit Bicarbonat) neutralisiert, ggf. trocknet, das Lösemittel entfernt und anschließend das entsprechende Produkt ggf. durch Destillation oder Kristallisation aufreinigt.

In der anschließenden Reaktionsstufe b) werden die Phenylalkylchloride der allgemeinen Formel (III) zuerst mit Natriumthiosulfat zu den entsprechenden Estern und anschließend mit Chlor zu einem Phenylalkylsulfonylchlorid der allgemeinen Formel (IV) 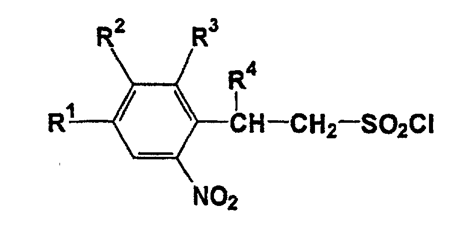 umgesetzt. Die Umsetzung mit Natriumthiosulfat erfolgt vorzugsweise in einem Lösemittelgemisch bestehend aus einem Alkohol und Wasser bei Temperaturen zwischen 50 und 100 °C, wobei insbesondere ein Konzentrationsverhältnis von 10 bis 100 mmol Phenylalkylchlorid pro 10 ml Alkohol/Wasser-Gemisch eingestellt wird. Als Alkohole haben sich vor allem Methanol und Ethanol bestens bewährt. Das Mengenverhältnis von Alkohol zu Wasser kann in weiten Grenzen variiert werden, doch hat es sich als vorteilhaft erwiesen, das Verhältnis von Alkohol zu Wasser auf annähernd 1 : 1 einzustellen.

Das Molverhältnis von Phenylalkylchlorid zu Natriumthiosulfat sollte zumindest 1 : 1 betragen, doch wird gemäß einer bevorzugten Ausführungsform mit einem deutlichen Überschuß an Natriumthiosulfat gearbeitet, der insbesondere 1,5 bis 2,5 mmol pro mmol Phenylalkylchlorid beträgt. Nach Abschluß der Reaktion, die in der Regel nach 10 bis 20 Std. beendet ist, wird das Lösemittel nach den üblichen Methoden ganz oder weitgehend entfernt und die entsprechenden Ester ohne weitere Isolierung oder Aufarbeitung mit Chlor zu den entsprechenden Phenylalkylsulfonylchloriden umgesetzt. Diese Chlorierung wird vorzugsweise in Wasser, einem Wasser/Essigsäure-Gemisch (bevorzugtes Mengenverhältnis 4 : 1 bis 2 : 1) oder einem Wasser/Dichlormethan-Gemisch (bevorzugtes Mengenverhältnis 2 : 1 bis 1:1) bei Temperaturen zwischen 0 und 10 °C durchgeführt, wobei mit einem großen Überschuß an Chlor gearbeitet werden kann. Vorzugsweise wird in dieser Stufe bei einer Konzentration von 5 bis 30 mmol Phenylalkylchlorid pro 100ml Solvens gearbeitet.

Nach der Chlorbehandlung (ca. 10 bis 30 Minuten) wird der Niederschlag abgetrennt und das Rohprodukt nach bekannten Methoden wie Kristallisation oder Säulenchromatographie aufgereinigt, wobei die entsprechenden Phenylalkylsulfonylchloride entweder als Feststoffe oder in Form von Ölen in sehr unterschiedlichen Ausbeuten anfallen.

Die Phenylalkylsulfonylchloride werden schließlich in der Reaktionsstufe c) mit den Nucleosiden der allgemeinen Formel (V) 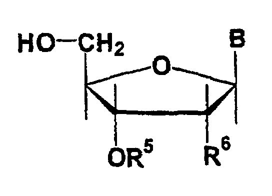 umgesetzt, wobei R⁵, R⁶ und B die oben angegebene Bedeutung besitzen.

Diese Umsetzung erfolgt vorzugsweise in einem Lösemittelgemisch bestehend aus Dichlormethan und Pyridin bei Temperaturen zwischen -60 und 0 °C. Das Mischungsverhältnis von Dichlormethan zu Pyridin ist relativ unkritisch, doch werden vorzugsweise 1 bis 3 Vol.-teile Dichlormethan pro Vol.-teil Pyridin eingesetzt.

Gemäß einer bevorzugten Ausführungsform wird das entsprechende Nucleosid (V), welches in Pyridin gelöst wurde, vorgelegt und eine Lösung des Phenylalkylsulfonylchlorids in Dichlormethan bei der jeweiligen Reaktionstemperatur zugetropft. Das Molverhältnis von Nucleosid zu Phenylalkylsulfonylchlorid kann hierbei entsprechend der Stöchiometrie auf ca. 1 : 1 eingestellt werden. Vorzugsweise wird jedoch das Phenylalkylsulfonylchlorid im Überschuß eingesetzt, und zwar in einer solchen Menge, daß das Molverhältnis Nucleosid zu Phenylalkylsulfonylchlorid 1 : 1 bis 1 : 2 beträgt. Schließlich kann auch die Konzentration des Nucleosids im Lösemittelgemisch weitgehend variiert werden, doch wird es bevorzugt auf 0,1 bis 3,0 mmol pro 10 ml Solvens eingestellt.

Nach erfolgter Umsetzung (Reaktionszeit ca. 1 bis 10 Std.) können die erfindungsgemäßen Nucleosid-Derivate nach bekannten Methoden isoliert bzw. gereinigt werden, wie z. B. Verdünnen mit Dichlormethan, Auswaschen der Salze mit Wasser, Trocknen der organischen Phase, Einengen der Lösung bzw. Kristallisation und anschließende Säulenchromatographie. Auf diese Weise können die entsprechenden Nucleosid-Derivate mit hoher Reinheit und in guten Ausbeuten (30 bis 65 %) erhalten werden.

Gemäß einer bevorzugten Ausführungsform kann man im Anschluß an die Reaktionsstufe b) in die 3'-Stellung der Nucleosid-Derivate mit R⁵ = H die Phosphitamid-Gruppe 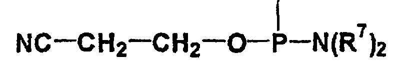 oder 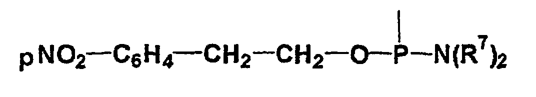 nach bekannten Methoden einführen. Üblicherweise erfolgt diese Umsetzung mit den entsprechenden Phosphinen in Gegenwart von 1H-Tetrazol als Aktivator in einem Lösemittelgemisch bestehend aus Dichlormethan und Acetonitril bei Temperaturen zwischen 0 und 25 °C. Vorzugsweise wird das Phosphin in zwei- bis dreifachem molaren Überschuß eingesetzt, während das Molverhältnis Phosphin zu 1H-Tetrazol auf 3 : ca. 1,0 eingestellt wird. Das Mengenverhältnis von Dichlormethan zu Acetonitril ist relativ unkritisch und beträgt vorzugsweise 1 : 1 bis 4 : 1.

Nach erfolgter Umsetzung (ca. 10 bis 20 Std.) kann das entsprechende Nucleosid wie in Stufe c) beschrieben aufgearbeitet werden.

Wie Bestrahlungsversuche mit polychromatischem Licht mit Wellenlänge > 289nm belegen, lassen sich die erfindungsgemäßen Nucleoside sehr rasch (t_{0,5} = ca. 1 bis 40 Minuten) und weitgehend entschützen (Ausbeuten bis zu 97%), so daß die besonderen Anforderungen an die Photolabilität der Schutzgruppen in hervorragender Weise erfüllt werden.

Aufgrund dieser besonderen Eigenschaften eignen sich die erfindungsgemäßen Nucleoside sehr gut für die Herstellung von Oligonucleotiden durch lichtgesteuerte Schutzgruppenabspaltung insbesondere auf festen Trägerplatten.

Die nachfolgenden Beispiele sollen die Erfindung näher erläutern.

### Beispiele

### Allgemeines:

### Lösemittel und Reagenzien

Lösemittel wurden destilliert oder nach den üblichen Methoden getrocknet. Für die Chromatographie wurden nur destillierte Lösungsmittel verwendet. Alle zu Synthesen verwendeten Chemikalien wurden in p.a. Qualität eingesetzt.

### Chromatographie

Die analytische Dünnschichtchromatographie wurde auf Fertigfolien der Fa. Merck mit Fluoreszenzmarker (Kieselgel 60, F₂₅₄) durchgeführt. Für die präparative Flash-Säulenchromatographie wurde Flash-Kieselgel der Fa. Baker verwendet. Es wurde mit einem Überdruck zwischen 0,25 bis 0,35 bar gearbeitet.

### UV/VIS-Absorptionsspektren

Die UV-Spektren wurden in Methanol (Uvasol, der Fa. Merck) mit einem Spektrometer der Fa. Perkin-Elmer, Modell Lambda 5, gemessen. Bei den Synthesevorschriften sind jeweils λ [nm] und (lg ε) angegeben. Schultern wurden in []-Klammern gesetzt.

### ¹H-NMR-Spektren

Zur Aufnahme der ¹H-NMR-Spektren diente ein 250 MHz-FT-Spektrometer, Modell AC 250, der Fa. Bruker. Die Spektren wurden auf das Protonensignal des Lösungsmittels (CDCl₃: 7,24, D₆-DMSO: 2,49) geeicht.

### Abkürzungen

LM = Lösemittel
EE = Essigsäureethylester
PE = Petrolether
TOL = Toluol
NPE = 2-(4-Nitrophenyl)ethyl-
NPES = 2-(2-Nitrophenyl)ethylsulfonyl-
NPPS = 2-(2-Nitrophenyl)propylsulfonyl-

### Herstellung der Phenylalkylchloride

### Beispiel 1

### 2-(2-Nitrophenyl)ethylchlorid

Zu 10,13 g 2-(2-Nitrophenyl)ethanol (60 mmol) in 36 ml abs. Toluol gibt man 2,1 ml abs. Pyridin und 21,62 g Thionylchlorid (13,3 ml, 0,18 mol). Nach 2 h am Rückfluß läßt man abkühlen und gießt auf Eis. Das Eiswasser wird mit 50 ml Chloroform versetzt und noch 2 x mit je 50 ml Chloroform extrahiert. Die vereinigten organischen Phasen neutralisiert man noch 2 x mit je 100 ml gesättigter Bicarbonat-Lösung. Nach dem Trocknen mit Na₂SO₄ wird abfiltriert und einrotiert. Nach Destillation im Hochvakuum erhält man 9,7 g (50 mmol, 87%) 2-(2-Nitrophenyl)ethylchlorid mit einem Siedepunkt von 66 bis 67 °C (0,001 mbar) als gelbes Öl.

DC (Kieselgel): R_{f} = 0,39 (PE/EE 9:1)
¹H-NMR (250 MHz, CDCl₃):
8,00 (dd, 1H, arom. H, o zu NO₂), 7,59 (t, 1H, arom. H), 7,45 (m, 2H, arom. H), 3,85 (t, 2H, α-CH₂), 3,38 (t, 2H, β-CH₂)
UV (MeOH), λ [nm] (lg ε):
204 (4,06), [216 (3,78)], 256 (3,69)

### Beispiel 2

### 2-(2-Chlor-6-nitrophenyl)ethylchlorid

Zu 8,8 g 2-(2-Chlor-6-nitrophenyl)ethanol (44 mmol) in 100 ml abs. Toluol tropft man 15,60 g Thionylchlorid (130 mmol) in 10 ml Toluol (abs.) schnell zu. Die Reaktionslösung wird 1 h am Rückfluß erhitzt. Nach dem Abkühlen wird auf Eis gegossen, mit 50 ml CH₂Cl₂ verdünnt und die H₂O-Phase noch 2 x mit je 50 ml CH₂Cl₂ extrahiert. Die vereinigten organischen Phasen trocknet man über Na₂SO₄ und rotiert ein. Man erhält 9,08 g (41 mmol, 94 %) 2-(2-Chlor-6-nitrophenyl)ethylchlorid als braunes Öl, das nach mehrtägiger Lagerung im Kühlschrank kristallisiert.

DC (Kieselgei): R_{f} = 0,71 (PE/EE 7:3)
Schmp.: < 25 °C
1H-NMR (250 MHz, CDCl₃):
7,76 (dd, 1H, arom. H), 7,66 (dd, 1H, arom. H), 7,38 (t, 1H, arom. H, m zu NO₂), 3,80 (m, 2H, α-CH₂), 3,44 (m, 2H, β-CH₂)
UV (MeOH), λ [nm] (lg ε):
212 (3,78), 252 (3,55), [286 (3,15)]

| Elementaranalyse: C₈H₇Cl₂NO₂ (220,1 g/mol) | | | |
|---|---|---|---|
| | C | H | N |
| ber. | 43,66 | 3,21 | 6,36 |
| gef. | 43,95 | 3,35 | 6,31 |

### Beispiel 3

### 2-(4-Chlor-2-nitrophenyl)ethylchlorid

Zu einer Lösung von 6,78 g 2-(4-Chlor-2-nitrophenyl)ethanol (33 mmol) in 100 ml abs. Toluol und 2,5 ml Pyridin tropft man schnell 12 g Thionylchlorid (7,4 ml, 100 mmol) in 10 ml Toluol (abs.). Man erhitzt 2 h am Rückfluß, läßt abkühlen, gießt auf 100 g Eis und versetzt mit 100 ml CH₂Cl₂. Die wäßrige Phase wird noch 2 x mit je 50 ml CH₂Cl₂ extrahiert. Die vereinigten organischen Phasen werden 2 x mit je 80 ml gesättigter Bicarbonat-Lösung neutralisiert, über Na₂SO₄ getrocknet, abfiltriert und einrotiert. Man erhält 7,12 g (32 mmol, 98 %) 2-(4-Chlor-2-nitrophenyl)ethylchlorid als braunes Öl, das nach einigen Tagen im Kühlschrank zu einem braunen Feststoff erstarrt.

DC (Kieselgel): R_{f} = 0,54 (PE/EE 9:1)
Schmp.: < 25 °C
¹H-NMR (250 MHz, CDCl₃):
8,01 (d, 1H, Hₐ), 7,57 (dd, 1H, H_{b}), 7,41 (d, 1H, H_{c}), 3,83 (t, 2H, α-CH₂), 3,35 (t, 2H, β-CH₂)
UV (MeOH), λ [nm] (lg ε):
214 (4,27), 254 (3,65), 293 (3,48)

| Elementaranalyse: C₈H₇Cl₂NO₂ (220,1 g/mol) | | | |
|---|---|---|---|
| | C | H | N |
| ber. | 43,67 | 3,21 | 6,37 |
| gef. | 43,72 | 3,14 | 6,15 |

### Beispiel 4

### 2-(2,4-Dinitrophenyl)ethylchlorid

20 g 2-(2,4-Dinitrophenyl)ethanol (94 mmol) werden in 120 ml abs. Toluol und 4 ml Pyridin gelöst. Man tropft schnell 34 g Thionylchlorid (21 ml, 282 mmol) in 20 ml Toluol, abs. zu. Nach 2 h am Rückfluß läßt man abkühlen und gießt auf Eis. Man versetzt mit 100 ml CH₂Cl₂ und extrahiert die wäßrige Phase noch 2 x mit je 50 ml CH₂Cl₂. Die vereinigten organischen Phasen werden über Na₂SO₄ getrocknet, abfiltriert und einrotiert. Man erhält 21,51 g (93 mmol, 98 %) 2-(2,4-Dinitrophenyl)ethylchlorid als braunes Öl.

DC (Kieselgel): R_{f} = 0,62 (PE/EE 7:3)
¹H-NMR (250 MHz, CDCl₃):
8,87 (d, 1H, Hₐ), 8,44 (dd, 1H, H_{b}), 7,71 (d, 1H, H_{c}), 3,90 (t, 2H; α-CH₂), 3,50 (t, 2H, β-CH₂)
UV (MeOH), λ [nm] (lg ε):
211 (4,43), 266 (4,22), [273 (4,16)]

### Beispiel 5

### 2-(2-Nitrophenyl)propylchlorid

Zu 6,2 g 2-(2-Nitrophenyl)propanol (34 mmol) in 90 ml abs. Toluol und 2 ml Pyridin tropft man 12,16 g Thionylchlorid (7,5 ml, 102 mmol) in 10 ml Toluol (abs.) schnell zu. Die Reaktionslösung wird 1 h am Rückfluß erhitzt. Nach dem Abkühlen wird auf 100 g Eis gegossen und mit 80 ml CH₂Cl₂ versetzt. Die H₂O-Phase extrahiert man noch 2 x mit je 80 ml CH₂Cl₂. Die vereinigten organischen Phasen werden mit 120 ml gesättigter Bicarbonat-Lösung neutralisiert, über Na₂SO₄ getrocknet und einrotiert. Man erhält 6,62 g (33 mmol, 98 %) 2-(2-Nitrophenyl)propylchlorid als braunes Öl.

DC (Kieselgel): R_{f} = 0,75 (PE/EE 7:3)
¹H-NMR (250 MHz, CDCl₃):
7,80 (dd, 1H, arom. H, o zu NO₂), 7,60 (m, 1H, arom. H), 7,44 (m, 2H, arom. H), 3,74 (m, 3H, α-CH₂, β-CH), 1,46 (d, 3H, CH₃)
UV (MeOH), λ [nm] (lg ε):
206 (3,79), [217 (4,05)], 251 (3,60)

| Elementaranalyse: C₉H₁₀ClNO₂ (199,6 g/mol) | | | |
|---|---|---|---|
| | C | H | N |
| ber. | 54,15 | 5,05 | 7,02 |
| gef. | 54,24 | 5,01 | 7,06 |

### Herstellung der Phenylalkylsulfonylchloride

### Beispiel 6

### 2-(2-Nitrophenyl) ethylsulfonylchlorid

3,7 g 2-(2-Nitrophenyl)ethylchlorid (20 mmol) werden mit 7,8 g Natriumthiosulfat-Pentahydrat (31 mmol) in 95 ml 50 %igem wäßrigem Methanol gelöst und 16 h am Rückfluß erhitzt. Man filtriert die Lösung nach dem Abkühlen und rotiert ein bis Niederschlag ausfällt. Es wird in einen 500ml Dreihalskolben umgefüllt, auf 10 °C abgekühlt und mit 100 g Eis versetzt. Man leitet 10 Min. einen starken Chlorstrom durch die Lösung. Die Temperatur sollte dabei nicht über 10 °C steigen. Es wird noch 1/2 h bei Raumtemperatur gerührt, so daß überschüssiges Chlor entweichen kann. Man saugt den Niederschlag über eine Glasfritte ab und trocknet ihn im Exsikkator. Zur Reinigung wird der Niederschlag in Chloroform gelöst und mit wenig Petrolether ausgefällt. Man erhält 3,2 g (13 mmol, 65 %) 2-(2-Nitrophenyl)ethylsulfonylchlorid als beige Kristalle.

### DC (Kieselgel): R_{f} = 0,65 (PE/EE 7:3)

Schmp.: 76 bis 77 °C (Lit.: 74 bis 75 °C)
¹H-NMR (250 MHz, CDCl₃):
8,09 (dd, 1H, arom. H, o zu NO₂), 7,69 (t, 1H, arom. H), 7,55 (m, 2H, arom. H), 4,10 (t, 2H, α-CH₂), 3,62 (t, 2H, β-CH₂)
UV (MeOH), λ [nm] (lg ε):
203 (4,00), 214 (3,75), 257 (3,62)

### Beispiel 7

### 2-(2-Chlor-6-nitrophenyl)ethylsulfonylchlorid

1,3 g 2-(2-Chlor-6-nitrophenyl)ethylchlorid (6 mmol) und 3,7 g Natriumthiosulfat-Pentahydrat (15 mmol) werden in 50 ml Ethanol/Wasser (1:1) 3 Tage am Rückfluß erhitzt. Die Reaktionslösung wird heiß filtriert und bis zur Trockene einrotiert. Man löst den Rückstand in H₂O und extrahiert 2 x mit CH₂Cl₂, um nicht umgesetztes Edukt zu entfernen. In die auf 3 °C gekühlte Wasserphase wird Chlor eingeleitet. Dabei muß darauf geachtet werden, daß die Temperatur nicht über 10 °C steigt. Man rührt noch 30 Min., damit überschüssiges Chlor entweichen kann. Der Niederschlag wird abgesaugt (geht äußerst schlecht, da es eine zähe, klebrige Masse ist). Reinigung über Flash-Chromatographie, das Rohprodukt wird in wenig CH₂Cl₂ gelöst auf die Säule aufgetragen, da es sich in Petrolether nicht vollständig löst (36 g Kieselgel, 3 x 10 cm, LM: PE/EE, kond. 9:1, Gradient: 200 ml PE/EE 9:1, 80 ml 7:1, 140 ml 6:1, 120 ml 5:1, 100 ml 4:1). Man erhält 400 mg (1,5 mmol, 25 %) 2-(2-Chlor-6-nitrophenyl)ethylsulfonylchlorid als gelblichen Feststoff.

DC (Kieselgel): R_{f} = 0,19 (PE/EE 9:1)
Schmp.: 75 bis 76 °C
¹H-NMR (250 MHz, CDCl₃):
7,88 (dd, 1H, arom. H), 7,74 (dd, 1H, arom. H), 7,51 (t, 1H, arom. H, m zu NO₂), 4,08 (t, 2H, α-CH₂), 3,63 (t, 2H, β-CH₂)
UV (MeOH), λ [nm] (lg ε):
210 (4,23), 255 (3,61), 269 (3,44)

| Elementaranalyse: C₈H₇Cl₂NO₄S (268,1 g/mol) | | | |
|---|---|---|---|
| | C | H | N |
| ber. | 33,82 | 2,48 | 4,93 |
| gef. | 34,11 | 2,50 | 5,10 |

### Beispiel 8

### 2-(4-Chlor-2-nitrophenyl)ethylsulfonylchlorid

5,02 g 2-(4-Chlor-2-nitrophenyl)ethylchlorid (22 mmol) und 8,89 g Natriumthiosulfat-Pentahydrat (36 mmol) werden in 80 ml 50 %igem wäßrigem Methanol gelöst und 15 h am Rückfluß erhitzt. Nach dem Abkühlen filtriert man ab und rotiert die Lösung auf 50 ml ein. Man füllt in einen 500 ml Dreihalskolben um und kühlt auf 10 °C ab, versetzt mit 25 ml Eisessig und 100g Eis. Nach 10 Min. Einleiten von Chlorgas (die Temperatur soll nicht über 10 °C steigen) wird noch 1/2 h bei Raumtemperatur gerührt, um überschüssiges Chlor entweichen zu lassen. Der entstandene zähe, klebrige Niederschlag wird abgesaugt, mit viel Wasser gewaschen und über Nacht im Exsikkator über NaOH getrocknet. Die restliche Substanz im Kolben wird in CH₂Cl₂ gelöst, über Na₂SO₄ getrocknet und einrotiert. Der getrocknete Niederschlag wird ebenfalls in CH₂Cl₂ aufgenommen, mit dem anderen Niederschlag vereinigt und einrotiert. Das Rohprodukt (5,636 g) wird auf Kieselgel aufgezogen und über Flash-Chromatographie gereinigt (150 g Kieselgel, 5 x 11 cm, LM: PE/EE, kond. 15:1, Gradient: 250 ml 15:1, 330 ml 10:1, 340 ml 7,5:1, 360 ml 5:1). Man erhält 3,38 g (12 mmol, 55 %) 2-(4-Chlor-2-nitrophenyl)ethylsulfonylchlorid als gelben Feststoff.

### DC (Kieselgel): R_{f} = 0,65 (PE/EE 7:3)

Schmp.: 59 bis 61 °C
¹H-NMR (250 MHz, CDCl₃):
8,11 (d, 1H, Hₐ), 7,63 (dd, 1H, H_{b}), 7,46 (d, 1H, H_{c}), 4,07 (t, 2H, α-CH₂), 3,59 (t, 2H, β-CH₂)
UV (MeOH), λ [nm] (lg ε):
214 (4,26), 255 (3,60), 300 (3,11)

| Elementaranalyse: C₈H₇Cl₂NO₄S (284,1 g/mol) | | | |
|---|---|---|---|
| | C | H | N |
| ber. | 33,82 | 2,48 | 4,93 |
| gef. | 34,25 | 2,47 | 4,97 |

### Beispiel 9

### 2-(2,4-Dinitrophenyl)ethylsulfonylchlorid

9,22 g 2-(2,4-Dinitrophenyl)ethylchlorid (40 mmol) und 14,89 g Natriumthiosulfat-Pentahydrat (60 mmol) werden in 180 ml 50 %igem wäßrigem Methanol gelöst und 16 h am Rückfluß erhitzt. Nach dem Abkühlen filtriert man ab und rotiert die Lösung auf die Hälfte des Volumens ein. Man füllt in einen 500 ml Dreihalskolben um, kühlt auf 10 °C ab und versetzt die Lösung mit 50 ml Eisessig und 150 g Eis. Nach 10 Min. Einleiten von Chlorgas (die Temperatur soll nicht über 10 °C steigen) wird noch 1/2 h bei Raumtemperatur gerührt, um überschüssiges Chlor entweichen zu lassen. Der entstandene zähe, klebrige Niederschlag wird abgesaugt und mit viel Wasser gewaschen. Die restliche Substanz im Kolben wird ebenso wie der abgesaugte Niederschlag in CH₂Cl₂ aufgenommen, über Na₂SO₄ getrocknet und einrotiert. Man erhält 7,34 g braunes Öl (25 mmol, 62 %), das noch verunreinigt ist. Zur weiteren Reinigung wird jeweils die Hälfte des Rohproduktes auf Kieselgel aufgezogen und über Flash-Chromatographie (70 g Kieselgel, 4 x 11 cm, LM: PE/EE, kond.: 15:1, Gradient: 200 ml 15:1, 330 ml 10:1, 340 ml 7,5:1, 350 ml 6:1, 360 ml 5:1) gereinigt. Man erhält insgesamt 4,37 g (15 mmol, 37 %) 2-(2,4-Dinitrophenyl)ethylsulfonylchlorid als gelben Feststoff.

DC (Kieselgel): R_{f} = 0,42 (PE/EE 7:3)
Schmp.: 79 bis 80 °C
¹H-NMR (250 MHz, CDCl₃):
8,96 (d, 1H, Hₐ), 8,50 (dd, 1H, H_{b}), 7,78 (d, 1H, H_{c}), 4,12 (t, 2H, α-CH₂), 3,73 (t, 2H, β-CH₂)
UV (MeOH), λ [nm] (lg ε):
239 (4,21), [255 (4,12)]

| Elementaranalyse: C₈H₇ClN₂O₆S (294,7 g/mol) | | | |
|---|---|---|---|
| | C | H | N |
| ber. | 32,61 | 2,39 | 9,51 |
| gef. | 32,80 | 2,31 | 9,15 |

### Beispiel 10

### 2-(2-Nitrophenyl)propylsulfonylchlorid

2 g 2-(2-Nitrophenyl)propylchlorid (10 mmol) und 3,75 g Natriumthiosulfat-Pentahydrat (15 mmol) werden in 50 ml 50 %igem wäßrigem Methanol gelöst und 15 h am Rückfluß erhitzt. Nach dem Abkühlen filtriert man ab und rotiert die Lösung bis zum Öl ein. Das Öl wird in einen 250 ml Dreihalskolben umgefüllt, auf 10 °C abgekühlt, mit 50 ml H2O, 25 ml Eisessig und 50 g Eis versetzt. Nach 10 Min. Einleiten von Chlorgas (die Temperatur soll nicht über 10 °C steigen) wird noch 1/2 h bei Raumtemperatur gerührt, um überschüssiges Chlor entweichen zu lassen. Man extrahiert die Reaktionslösung 1 x mit 200 ml und 2 x mit je 75 ml Ether. Die vereinigten Etherphasen werden mit je 100 ml 5 %iger Natriumbisulfit-Lösung und H₂O gewaschen, über Na₂SO₄ getrocknet und einrotiert. Das Rohprodukt (1,03 g) wird auf Kieselgel aufgezogen und über Flash-Chromatographie gereinigt (34g Kieselgel, 3 x 10 cm, LM: PE/EE, kond. 15:1, Gradient: 250 ml 15:1, 165 ml 10:1, 170 ml 7,5:1, 180 ml 5:1). Man erhält 458 mg Edukt 2-(2-Nitrophenyl)propylchlorid und 423 mg (1,6 mmol, 16 %) 2-(2-Nitrophenyl)propylsulfonylchlorid als rötliches Öl.

DC (Kieselgel) R_{f} = 0,51 (PE/EE 7:3)
¹H-NMR (250 MHz, CDCl₃):
7,91 (dd, 1H, arom. H, o zu NO₂), 7,74 (m, 1H, arom. H), 7,47 (m, 2H, arom. H), 4,19 (m, 2H, α-CH₂), 3,96 (m, 1H, β-CH), 1,63 (d, 3H, CH₃)
UV (MeOH), λ[nm] (lg ε):
204 (4,15), [216 (3,94)], 252 (3,64)

| Elementaranalyse: C₉H₁₀Cl₂NO₄S (263,7 g/mol) | | | |
|---|---|---|---|
| | C | H | N |
| ber. | 40,99 | 3,82 | 5,31 |
| gef. | 41,40 | 3,77 | 5,08 |

### Herstellung der Nucleosid-Derivate

### Beispiel 11

### N⁶-NPEOC-5'-O-[2-(2-nitrophenyl)ethylsulfonyl]-2'-desoxyadenosin

Zu 438 mg N⁶-NPEOC-2'-desoxyadenosin (1 mmol, 3 x mit je 4 ml abs. Pyridin koevaporiert) in 3,5 ml abs. Pyridin wird bei -45 °C 304 mg 2-(2-Nitrophenyl)ethylsulfonylchlorid (1,2 mmol) in 3,5 ml abs. CH₂Cl₂ innerhalb von 20 Min. zugetropft. Nach 4 h Rühren bei -40 bis -20 °C erhöht man die Temperatur 2 h auf -15 bis -5 °C und läßt sie dann noch 1 1/2 h ansteigen, bis 0 °C erreicht sind. Nach insgesamt 7 1/2 h wird die Lösung mit 15 ml H₂O und 15 ml CH₂Cl₂ versetzt. Man extrahiert die H₂O-Phase noch 2 x mit je 20 ml CH₂Cl₂ und trocknet die vereinigten organischen Phasen über Na₂SO₄. Man filtriert ab, rotiert ein und koevaporiert 3 x mit Toluol und 1 x mit MeOH. Das erhaltene Rohprodukt wird durch Flash-Chromatographie (37 g Kieselgel, 4 x 10 cm, LM: CH₂Cl₂/MeOH, kond.:100:1, Gradient: 80 ml 100:1, je 100 ml 100:2, 100:3, 100:4 und 100:5) gereinigt. Man erhält 174 mg (0,2 mmol, 20 %) N⁶-NPEOC-3',5'-di-O-[2-(2-nitrophenyl)ethylsulfonyl]-2'-desoxyadenosin und 219 mg (0,35 mmol, 35 %) N⁶-NPEOC-5'-O-[2-(2-nitrophenyl)ethylsulfonyl]-2'-desoxyadenosin als farblose Schäume.

DC (Kieselgel): R_{f} = 0,33 (Tol/EE/MeOH 5:4:1)
¹H-NMR (250 MHz, D₆-DMSO):
10,57 (s, 1H, NH), 8,59 (2 x s, 2H, H-C(8), H-C(2)), 8,15 (d, 2H, arom. H NPEOC, o zu NO₂), 7,96 (d, 1H, arom. H NPES, o zu NO₂), 7,61 (d, 2H, arom. H NPEOC, m zu NO₂), 7,47 (m, 3H, arom. H NPES), 6,48 (t, 1H, H-C(1')), 5,62 (d, 1H, OH-C(3')), 4,44 (m, 5H, H-C(3'), 2 x α-CH₂ NPE), 4,10 (m, 1H, H-C(4')), 3,62 (m, 2H, H-C(5')), 3,14 (m, 4H, 2 x β-CH₂ NPE), 2,87 (m, 1H, H-C(2')), 2,38 (m, 1H, H-C(2'))
UV (MeOH), λ [nm] (lg ε):
206 (4,56), 266 (4,46), 274 [(4,39)]

| Elementaranalyse: C₂₇H₂₇N₇O₁₁S x 1/2 H₂O (666,6 g/mol) | | | |
|---|---|---|---|
| | C | H | N |
| ber. | 48,64 | 4,23 | 14,71 |
| gef. | 48,63 | 4,11 | 14,31 |

### Beispiel 12

### 5'-O-[2-(2-Chlor-6-nitrophenyl)ethylsulfonyl]-N⁶-NPEOC-2'-desoxyadenosin

Zu 355 mg N⁶-NPEOC-2'-desoxyadenosin (0,8 mmol, 3 x mit je 4 ml abs. Pyridin koevaporiert) in 4 ml abs. Pyridin wird bei -45 °C 290 mg 2-(2-Chlor-6-nitrophenyl)ethylsulfonylchlorid (1,02 mmol) in 4 ml abs. CH₂Cl₂ innerhalb von 20 Min. zugetropft. Nach 4 h Rühren bei -40 bis -30 °C läßt man die Temperatur 2 1/2 h ansteigen, bis 0 °C erreicht sind. Nach insgesamt 6 1/2h wird die Lösung mit 15 ml H₂O und 15 ml CH₂Cl₂ versetzt und die H₂O-Phase noch 3 x mit je 15 ml CH₂Cl₂ extrahiert. Man trocknet die vereinigten organischen Phasen über Na₂SO₄, filtriert ab, rotiert ein und koevaporiert 3 x mit Toluol und 1 x mit MeOH. Das erhaltene Rohprodukt wird durch Flash-Chromatographie (32 g Kieselgel, 3 x 10 cm, LM: CH₂Cl₂/MeOH, kond.: 100:1, Gradient: 100 ml 100:1, je 100 ml 100:2, 100:3, 100:4 und 100:5) gereinigt. Man erhält 214 mg (0,3 mmol, 39 %) 5'-O-[2-(2-Chlor-6-nitrophenyl) -ethylsulfonyl]-N⁶-NPEOC-2'-desoxyadenosin als farblosen Schaum.

DC (Kieselgel): R_{f} = 0,36 (Tol/EE/MeOH 5:4:1)
¹H-NMR (250 MHz, D₆-DMSO):
10,56 (s, 1H, NH), 8,58 (s, 2H, H-C(8), H-C(2)), 8,15 (d, 2H, arom. H NPEOC, o zu NO₂), 7,94 (d, 1H, arom. H NPES), 7,81 (d, 1H, arom. H NPES), 7,56 (d, 3H, 2 arom. H NPEOC, m zu NO₂, 1 arom. H NPES, m zu NO₂), 6,47 (t, 1H, H-C(1')), 5,62 (d, 1H, OH-C(3')), 4,46 (m, 5H, H-C(3')), 2 x α-CH₂ NPE), 4,12 (m, 1H, H-C(4')), 3,52 (m, 2H, H-C(5')), 3,21 (m, 2H, β-CH₂ NPE), 3,10 (m, 2H, β-CH₂ NPE), 2,88 (m, 1H, H-C(2')), 2,38 (m, 1H, H-C(2'))
UV (MeOH), λ [nm] (lg ε):
210 (4,62), 266 (4,43), [272 (4,39)]

| Elementaranalyse: C₂₇H₂₆ClN₇O₁₁S x 1/2 H₂O (701,1 g/mol) | | | |
|---|---|---|---|
| | C | H | N |
| ber. | 46,25 | 3,74 | 13,98 |
| gef. | 46,33 | 3,79 | 13,48 |

### Beispiel 13

### 5'-O-[2-(4-Chlor-2-nitrophenyl)ethylsulfonyl]-N⁴-NPEOC-2'-desoxycytidin

Zu 250 mg N⁴-NPEOC-2'-desoxycytidin (0,59 mmol, 3 x mit je 4 ml abs. Pyridin koevaporiert) in 2,5 ml abs. Pyridin tropft man bei 0 °C innerhalb von 45 Min. 253 mg 2-(4-Chlor-2-nitrophenyl)ethylsulfonylchlorid (0,89 mmol) in 2,5 ml abs. CH₂Cl₂ zu. Nach 3 3/4 h Rühren bei 0 °C wird die Lösung mit 10 ml H₂O und 15 ml CH₂Cl₂ versetzt. Man extrahiert die H₂O-Phase noch 1 x mit 15 ml CH₂Cl₂. Die vereinigten organischen Phasen werden über Na₂SO₄ getrocknet, abfiltriert, einrotiert und 3 x mit Toluol und 2 x mit MeOH koevaporiert. Das erhaltene Rohprodukt (942 mg) wird durch Flash-Chromatographie (37 g Kieselgel), 3 x 10 cm, LM: CH₂Cl₂/MeOH, kond.: CH₂Cl₂, Gradient: 100 ml CH₂Cl₂, je 200 ml 100:1, 100:2, 100:3 und 100 ml 100:4) gereinigt. Man erhält 214 mg (0,32 mmol, 34 %) 5'-O-[2-(4-Chlor-2-nitrophenyl)ethylsulfonyl]-N⁴-NPEOC-2'-desoxycytidin als farblosen Schaum. Die erhaltenen Mischfraktionen werden noch einmal chromatographiert(5 g Kieselgel, 1 x 12 cm, LM: CH₂Cl₂/MeOH, kond.: CH₂Cl₂, Gradient: 70 ml CH₂Cl₂, je 100 ml 100:5, 100:1 und 50 ml 100:2). Man erhält 147 ml (0,16 mmol, 27 %) 3'-O-[2-(4-Chlor-2-nitrophenyl)ethyl-sulfonyl]-N⁴-NPEOC-2'-desoxycytidin und 24 mg (0,04 mmol, 6 %) 3',5'-Di-O-[2-(4-chlor-2-nitrophenyl)ethylsulfonyl]-N⁴-NPEOC-2'-desoxycytidin ebenfalls als farblose Schäume.

DC (Kieselgel): R_{f} = 0,43 (Tol/EE/MeOH 5:4:1)
¹H-NMR (250 MHz, D⁶-DMSO):
10,78 (s, 1H, NH), 8,15 (d, 2H, arom. H NPEOC, o zu NO₂), 8,08 (d, 1H, Hₐ), 8,01 (d, 1H, H-C(6)), 7,75 (dd, 1H, H_{b}), 7,59 (d, 3H, 2 arom. H NPEOC, m zu NO₂, H_{c}), 6,94 (d, 1H, H-C(5)), 6,14 (t, 1H, H-C(1')), 5,52 (d, 1H, OH-C(3')), 4,43 (d, 2H, α-CH₂ NPE), 4,34 (t, 2H, α-CH₂ NPE), 4,21 (m, 1H, H-C(3')), 4,03 (m, 1H, H-C(4')), 3,73 (m, 2H, H-C(5')), 3,25 (m, 2H, β-CH₂ NPE), 3,07 (m, 2H, β-CH₂ NPE), 2,27 (m, 1H, H-C(2')), 2,12 (m, 1H, H-C(2'))
UV (MeOH), λ [nm] (lg ε):
[207 (4,61)], 212 (4,65), 244 (4,36), [269 (4,28)]

| Elementaranalyse: C₂₆H₂₆ClN₅O₁₂S (668,0 g/mol) | | | |
|---|---|---|---|
| | C | H | N |
| ber. | 46,75 | 3,92 | 10,48 |
| gef. | 46,82 | 3,97 | 10,16 |

### Beispiel 14

### 5'-O-[2-(2,4-Dinitrophenyl)ethylsulfonyl]-N²-NPEOC-O⁶-NPE-2'-desoxyguanosin

Zu 391 mg N²-NPEOC-O⁶-NPE-2'-desoxyguanosin (0,65 mmol, 3 x mit je 8 ml abs. Pyridin koevaporiert) in 3 ml abs. Pyridin wird bei -50 °C 381 mg 2-(2,4-Dinitrophenyl)ethylsulfonylchlorid (1,3 mmol) in 3 ml abs. CH₂Cl₂ innerhalb von 40 Min. zugetropft. Nach 4 h Rühren bei -50 bis -30 °C und 2 1/2h bei -30 bis -15 °C versetzt man die Lösung mit 15 ml H₂O und 15 ml CH₂Cl₂. Die H₂O-Phase wird noch 1 x mit 20 ml CH₂Cl₂ extrahiert und die vereinigten organischen Phasen über Na₂SO₄ getrocknet. Man filtriert ab, rotiert ein und koevaporiert 3 x mit Toluol und 1 x mit MeOH. Das erhaltene Rohprodukt (730 mg) wird durch Flash-Chromatographie (33 g Kieselgel, 3 x 9 cm, LM: CH₂Cl₂/MeOH, kond.: CH₂Cl₂, Gradient: 100 ml CH₂Cl₂, je 200 ml 100:0,7, 100:1,4, 100:2, 100:3 und 100:4) gereinigt. Man erbält 141 mg (0,12 mmol, 19 %) noch leicht verunreinigtes 3',5'-Di-O-[2-(2,4-dinitrophenyl)ethyl- sulfonyl]-N²-NPEOC-O⁶-NPE-2'-desoxyguanosin und 339 mg (0,39 mmol, 60 %) 5'-O-[2-(2,4-Dinitrophenyl)ethylsulfonyl]-N²-NPEOC-O⁶-NPE-2'-desoxy- guanosin als leicht gelbliche Schäume.

DC (Kieselgel): R_{f} = 0,46 (Tol/EE/MeOH 5:4:1)
¹H-NMR (250 MHz, D₆-DMSO):
10,31 (s, 1H, NH), 8,64 (d, 1H, Hₐ), 8,34 (dd, 1H, H_{b}), 8,29 (s, 1H, H-C(8)), 8,15 (d, 4H, arom. H NPE, o zu NO₂), 7,62 (d, 4H, arom. H NPE, m zu NO₂) 7,57 (s, 1H, H_{c}), 6,33 (t, 1H, H-C(1')), 5,53 (d, 1H, OH-C(3')), 4,68 (t, 2H, α-CH₂ NPE), 4,48 (m, 3H, α-CH₂ NPE, H-C(3')), 4,33 (t, 2H, α-CH₂ NPE), 4,06 (m, 1H, H-C(4')), 3,66 (m, 2H, H-C(5')), 3,24 (m, 4H, 2 x β-CH₂ NPE), 3,08 (t, 2H, β-CH₂ NPE), 2,89 (m, 1H, H-C(2')), 2,26 (m, 1H, H-C(2'))
UV (MeOH), λ [nm] (lg ε) :
[206 (4,70)], 214 (4,74), [254 (4,60)], 265 (4,63)

| Elementaranalyse: C₃₅H₃₃N₉O₁₆S x 1/2 H₂O (876,8 g/mol) | | | |
|---|---|---|---|
| | C | H | N |
| ber. | 47,94 | 3,79 | 14,37 |
| gef. | 48,27 | 3,65 | 14,00 |

### Beispiel 15

### 5'-O-[2-(2-Nitrophenyl)propylsulfonyl]-thymidin

242 mg Thymidin (1 mmol, 3 x mit je 5 ml abs. Pyridin koevaporiert) werden in 2,5 ml abs. Pyridin gelöst und auf -60 °C gekühlt. Dazu tropft man innerhalb von 10 Min. 396 mg 2-(2-Nitrophenyl)propylsulfonylchlorid (1,5 mmol) in 2,5 ml abs. CH₂Cl₂. Nach 4 h Rühren bei einer Temperatur zwischen -60 und -30°C läßt man die Temperatur auf -15 °C ansteigen. Die Reaktion wird nach insgesamt 6 1/4 h mit je 15 ml H₂O und CH₂Cl₂ versetzt und die wäßrige Phase noch 4 x mit je 15 ml CH₂Cl₂ extrahiert. Die vereinigten organischen Phasen werden über Na₂SO₄ getrocknet, abfiltriert und einrotiert. Man koevaporiert noch 3 x mit Toluol und 1 x mit Methanol. Das Rohprodukt (600 mg) wird über Flash-Chromatographie (39 g Kieselgel, 3 x 11 cm, LM: CH₂Cl₂/MeOH, kond.: CH₂Cl₂, Gradient: 100 ml CH₂Cl₂, je 150 ml 100:1, 100:2, 100:3 und 100:4) gereinigt. Man erhält 290 mg (0,62 mmol, 62 %) 5'-O-[2-(2-Nitrophenyl)propyl- sulfonyl]-thymidin als farblosen Schaum. Die erhaltenen Mischfraktionen werden durch eine weitere Säule (4,5 g Kieselgel, 1 x 11 cm, LM: CH₂Cl₂/MeOH, kond: CH₂Cl₂, Gradient: 100 ml CH₂Cl₂, je 50 ml 100:0,5, 100:1 und 100:2) gereinigt. Es werden 128 mg (0,18 mmol, 18 %) 3',5'-Di-O-[2-(2-nitrophenyl)propylsulfonyl]-thymidin als farbloser Schaum und 26 mg (0,06 mmol, 6 %) 3'-O-[2-(2-Nitrophenyl)propylsulfonyl]-thymidin als leicht rötlicher Schaum isoliert.

DC (Kieselgel): R_{f} = 0,39 (Tol/EE/MeOH 5:4:1)
¹H-NMR (250 MHz, D₆-DMSO):
11,32 (d, 1H, NH), 7,84 bis 7,64 (m, 3H, arom. H NPPS), 7,44 (m, 2H, 1 arom. H NPPS, H-C(6)), 6,16 (q, 1H, H-C(1')), 5,45 (q, 1H, OH-C(3')), 4,32 bis 4,19 (m, 2H, H-C(5')), 4,06 (m, 1H, H-C(3')), 3,84 (d, 3H, a-CH₂ NPPS, H-C(4')), 3,71 (m, 1H, β-CH NPPS), 2,09 (m, 2H, H-C(2')), 1,73 (s, 3H, CH₃ Thymidin), 1,38 (dd, 3H, CH₃ NPPS)
UV (MeOH), λ [nm] (lg ε):
205 (4,28), [217 (4,08)], 262 (4,07)

| Elementaranalyse (Mol. Gew.): C₁₉H₂₃N₃O₉S (469,5 g/mol) | | | |
|---|---|---|---|
| | C | H | N |
| ber. | 48,61 | 4,94 | 8,95 |
| gef. | 48,69 | 4,95 | 8,71 |

### Bestrahlungsexperimente

### 1. Durchführung

Die entsprechend geschützten Nucleosid-Derivate wurden mit Hilfe einer Apparatur bestrahlt, die aus einer Hg-Höchstdrucklampe (200 W), einem IR-Filter (Wasser), einem Shutter (automatischer Verschluß zur exakten Regelung der Bestrahlungsdauer), einem Standard-Interferenzfilter (Filter 1) mit einem schmalen Bereich um die Wellenlänge 365 nm, einer Sammellinse sowie einem auf ca. 17 °C thermostatisierten Küvettenhalter bestand. Um die Überhitzung von Filter 1 zu verhindern, wurde ggf. zwischen Shutter und Filter 1 noch ein Breitbandfilter UG1 (Filter 2) installiert. Bei den Bestrahlungsversuchen wurde Licht der Wellenlänge 365 nm verwendet, damit nur die Schutzgruppen und nicht die heterocyclischen Basen angeregt werden. Die Bestrahlung erfolgte in Quarzküvetten (3,5 ml) mit je 3 ml Lösung (Ausgangskonzentration 0,1 bzw. 0,025 mmol/l). Nach erfolgter Bestrahlung wurden der Küvette zwei Proben entnommen und mit Hilfe eines HPLC-Systems analysiert.

Das HPLC-System der Fa. Merck-Hitachi bestand aus folgenden Geräten: Pumpe L-7100, Auto-Sampler L-7200, UV/VIS-Detektor (Detektionswellenlänge 260 nm) L-7420 und Interface D-7000. Als Säule wurde eine LICHROSORB RP 18 der Fa. Merck verwendet. Die Steuerung erfolgte mit einem Computer der Fa. Compaq durch den HSM-Manager.

Zur Chromatographie wurde der folgende Gradient (Lösemittel: Wasser und Acetonitril) verwendet (s. Tabelle 1).

**Tabelle 1**

| Gradient | | | | |
|---|---|---|---|---|
| Zeit [min] | H₂O | H₂O/MeCN (1:1) [%] | MeCN [%] | Fluß |
| 0 | 100 | 0 | 0 | 1 |
| 3 | 100 | 0 | 0 | 1 |
| 10 | 0 | 100 | 0 | 1 |
| 25 | 0 | 0 | 100 | 1 |
| 30 | 0 | 100 | 0 | 1 |
| 33 | 100 | 0 | 0 | 1 |
| 38 | 100 | 0 | 0 | 1 |

In den erhaltenen Chromatogrammen konnten die Abnahme des Eduktes (5'-O-geschütztes Nucleosid) und die Zunahme des Produktes (5'-O-entschütztes Nucleosid) verfolgt werden. Die Auswertung erfolgte dabei über die Fläche der einzelnen Peaks. Als Referenz wurde die Lösung des zu bestrahlenden Nucleosids zum Zeitpunkt 0 Min. (d. h. vor der Bestrahlung) eingespritzt und die Fläche des erhaltenen Peaks als 100 % Edukt angesehen. Gleichermaßen wurde für das Produkt verfahren: Es wurde die Peakfläche einer 0,1 bzw. 0,025 mmolaren Lösung bestimmt und als 100 % gesetzt. Die jeweiligen Flächen der Produkte und Edukte der anderen Zeitpunkte wurden auf diese Referenswerte bezogen.

Aus den so erhaltenen Kurven (Konz. in % gegen die Zeit aufgetragen) wurden folgende Werte abgelesen:
t_{H}: Halbwertszeit: der Zeitpunkt, an dem die Hälfte des Eduktes umgesetzt war
Konz. t_{H}: Konzentration des Produktes bei der Halbwertszeit
Konz. t_{end}: Konzentration des Produktes am letzten untersuchten Zeitpunkt. Meistens wurde dieser Zeitpunkt so gelegt, daß das Edukt nicht mehr nachweisbar war.

Die Ergebnisse der Bestrahlungsexperimente sind in Tabelle 2 zusammengefaßt.

Wie man aus Tabelle 2 ersieht, variieren die Halbwertszeiten bei den verschiedenen Nucleosiden relativ stark. Während das 5'-O-Phenylpropylsulfonyl-thymidin-Derivat (Beispiel 15) mit 49 Sekunden die kürzeste Halbwertszeit aufweist, beträgt diese beim 5'-O-[2-(2-Chlor-6-nitrophenyl)ethylsulfonyl]-geschützten 2'-Desoxyadenosin (Beispiel 12) 42 Minuten.

Was die Ausbeuten der entschützten Nucleoside betrifft, so läßt Tabelle 2 erkennen, daß beim 5'-O-[2-(2-Chlor-6-nitrophenyl)ethylsulfonyl]geschützten 2'-Desoxyadenosin (Beispiel 11) diese mit 97 % am höchsten liegt, während sie bei den anderen Nucleosid-Derivaten Werte zwischen ca. 50 und 85 % annimmt.

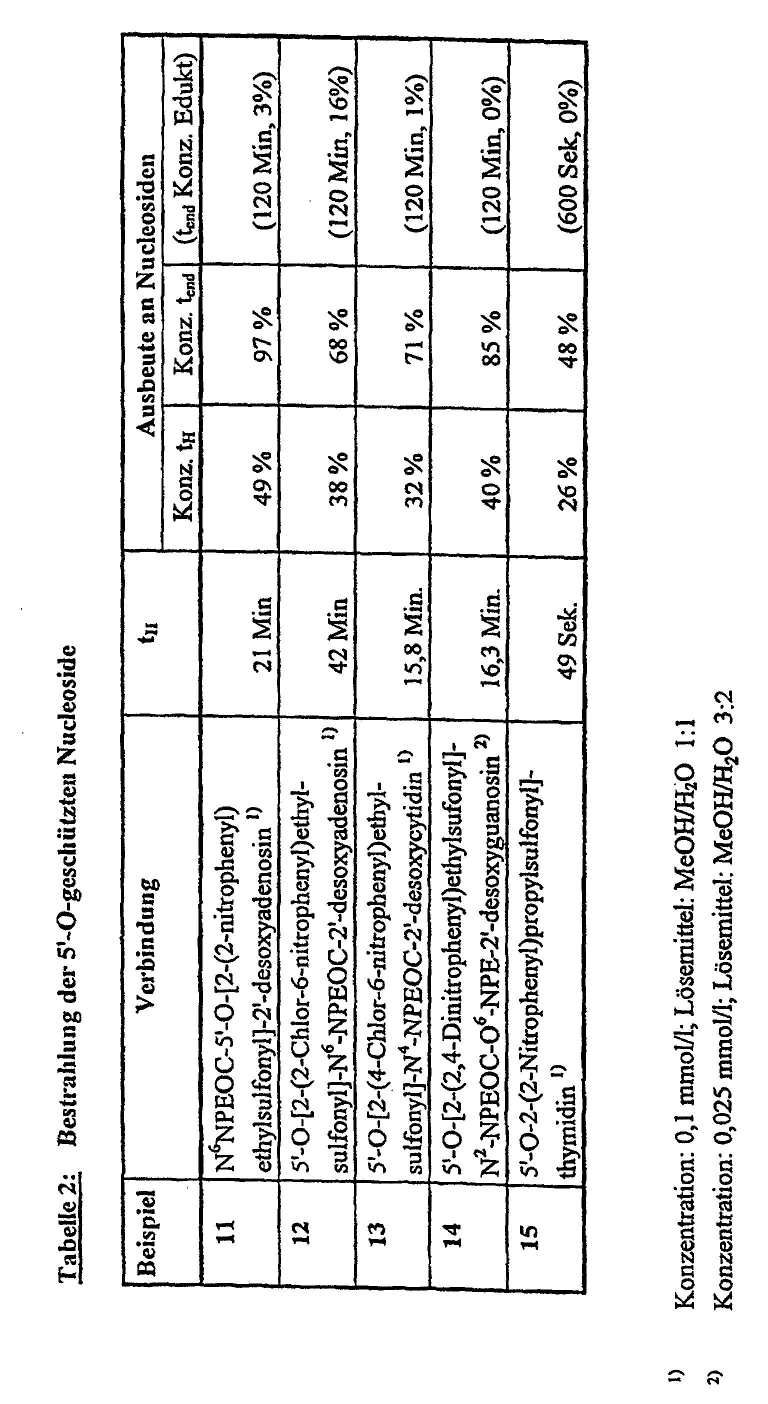

## Patentansprüche

1. Nucleosid-Derivate mit photolabilen Schutzgruppen der allgemeinen Formel (I) 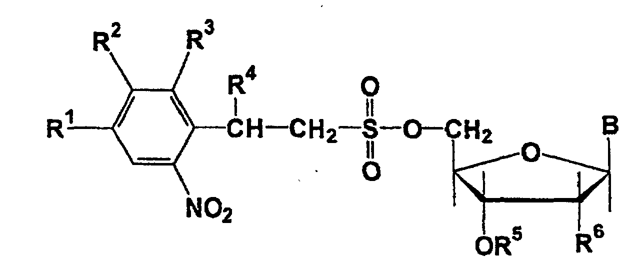 in der
R¹ = H, NO₂, CN, OCH₃, Halogen oder Alkyl oder Alkoxyalkyl mit 1 bis 4 C-Atomen
R² = H, OCH₃
R³ = H, F, Cl, Br, NO₂ oder ein aliphatischer Acylrest mit 2 bis 5 C-Atomen
R⁴ = H, Halogen, OCH₃, ein Alkylrest mit 1 bis 4 C-Atomen oder ein ggf. substituierter Arylrest
R⁵ = H oder eine übliche funktionelle Gruppe zur Herstellung von Oligonucleotiden
R⁶ = H, OH, Halogen oder XR⁸, wobei X = O oder S und R⁸ eine in der Nucleotidchemie übliche Schutzgruppe darstellt
B = Adenin, Cytosin, Guanin, Thymin, Uracil, 2,6-Diaminopurin-9-yl, Hypoxanthin-9-yl, 5-Methylcytosin-1-yl, 5-Amino-4-imidazolcarbonsäureamid-1-yl oder 5-Amino-4-imidazolcarbonsäureamid-3-yl, wobei im Falle von B = Adenin, Cytosin oder Guanin die primäre Aminofunktion ggf. eine permanente Schutzgruppe aufweist.

2. Nucleosid-Derivate nach Anspruch 1, **dadurch gekennzeichnet, daß** im Falle von R⁴ ≠ H R¹ = R² = R³ = H ist.

3. Nucleosid-Derivate nach den Ansprüchen 1 und 2, **dadurch gekennzeichnet, daß** R⁴ einen Methylrest darstellt.

4. Nucleosid-Derivate nach den Ansprüchen 1 bis 3, **dadurch gekennzeichnet, daß** R⁵ eine Phosphitamidgruppe der Formel 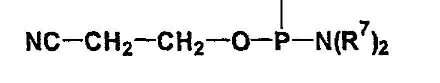 oder 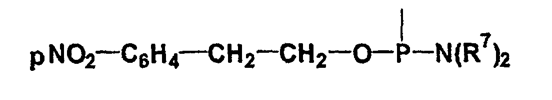 darstellt, wobei die R⁷-Gruppen gleich oder verschieden sind und lineare oder verzweigte Alkylreste mit 1 bis 4 C-Atomen bedeuten.

5. Nucleosid-Derivate nach Anspruch 4, **dadurch gekennzeichnet, daß** R⁷ einen Ethyl- oder Isopropylrest darstellt.

6. Nucleosid-Derivate nach den Ansprüchen 1 bis 5, **dadurch gekennzeichnet, daß** R⁶ eine Gruppe XR⁸ ist und R⁸ im Fall von X = O eine Alkyl-, Alkenyl-, Acetal- oder Silylether-Schutzgruppe oder im Fall von X = S eine Alkyl-Schutzgruppe darstellt.

7. Nucleosid-Derivate nach Anspruch 6, **dadurch gekennzeichnet, daß** als R⁶ ein O-Methyl- oder O-Ethylrest, ein O-Allylrest, ein O-Tetrahydropyranyl- bzw. O-Methoxytetrahydropyranyl-Rest oder ein O-t-Butyldimethylsilyl-Rest eingesetzt wird.

8. Nucleosid-Derivate nach den Ansprüchen 1 bis 7, **dadurch gekennzeichnet, daß** man im Falle von B = Adenin, Cytosin oder Guanin als permanente Schutzgruppe Phenoxyacetyl- oder Dimethylformamidino-Reste einsetzt.

9. Nucleosid-Derivate nach den Ansprüchen 1 bis 7, **dadurch gekennzeichnet, daß** man im Falle von B = Adenin als permanente Schutzgruppe Benzoyl- oder p-Nitrophenylethoxycarbonyl-(p-NPEOC)-Reste verwendet.

10. Nucleosid-Derivate nach den Ansprüchen 1 bis 7, **dadurch gekennzeichnet, daß** im Falle von B = Guanin die permanente Schutzgruppe Isobutyroyl-, p-Nitrophenylethyl-(p-NPE)- oder p-NPEOC-Reste darstellen.

11. Nucleosid-Derivate nach den Ansprüchen 1 bis 7, **dadurch gekennzeichnet, daß** man im Falle von B = Cytosin als permanente Schutzgruppe Benzoyl- oder p-NPEOC-Reste einsetzt.

12. Nucleosid-Derivate nach den Ansprüchen 1 bis 11, **dadurch gekennzeichnet, daß** als Halogenatom in R¹ oder R⁶ ein Fluor-, Chlor- oder Bromatom eingesetzt wird.

13. Verfahren zur Herstellung von Nucleosid-Derivaten nach den Ansprüchen 1 bis 12 in mindestens drei Stufen, **dadurch gekennzeichnet, daß** man
a) einen Alkohol der allgemeinen Formel (II) 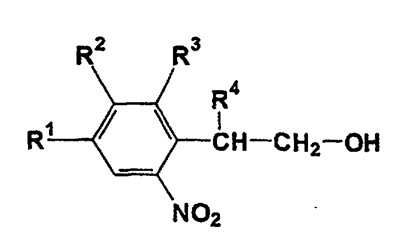 in der R¹, R², R³, R⁴ die oben angegebene Bedeutung besitzen, mit Thionylchlorid umsetzt und anschließend
b) das in Stufe a) gebildete Phenylalkylchlorid der allgemeinen Formel (III) 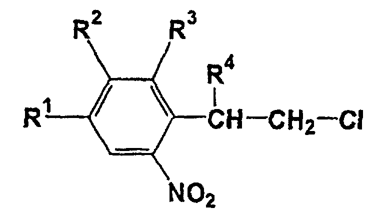 zuerst mit Natriumthiosulfat und anschließend mit Chlor zu einem Phenylalkylsulfonylchlorid der allgemeinen Formel (IV) 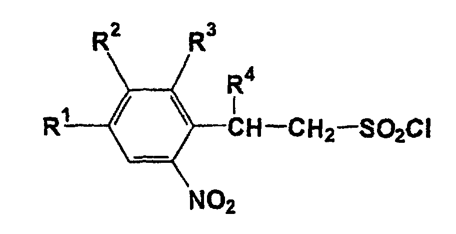 umwandelt und anschließend
c) das in Stufe b) gebildete Phenylalkylsulfonylchlorid mit Nucleosiden der allgemeinen Formel (V) 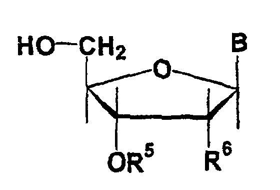 in der R⁵, R⁶ und B die oben angegebene Bedeutung besitzen, reagieren läßt und ggf. anschließend
d) in der 3'-Stellung der Nucleosid-Derivate mit R⁵ = H die Phosphitamid-Gruppe 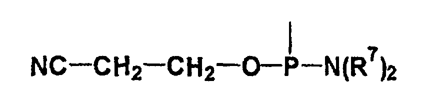 oder 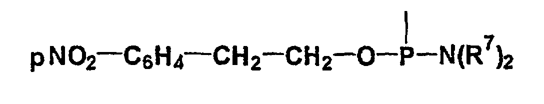 nach bekannten Methoden einführt.

14. Verfahren nach Anspruch 13, **dadurch gekennzeichnet, daß** man Stufe a) in einem unpolaren organischen Lösemittel bei Temperaturen zwischen 50 und 120 °C ggf. in Gegenwart einer Base durchführt.

15. Verfahren nach den Ansprüchen 13 und 14, **dadurch gekennzeichnet, daß** man in Stufe a) das Thionylchlorid in zwei- bis fünffachem Überschuß bezogen auf die Alkoholkomponente einsetzt.

16. Verfahren nach den Ansprüchen 13 bis 15, **dadurch gekennzeichnet, daß** man in Stufe a) als unpolares organisches Lösemittel Toluol verwendet.

17. Verfahren nach den Ansprüchen 13 bis 16, **dadurch gekennzeichnet, daß** man in Stufe a) als Base Pyridin einsetzt.

18. Verfahren nach Anspruch 17, **dadurch gekennzeichnet, daß** man das Pyridin in einer Menge von 2 bis 10 Vol.-% bezogen auf das eingesetzte Toluol verwendet.

19. Verfahren nach den Ansprüchen 13 bis 18, **dadurch gekennzeichnet, daß** die Konzentration der Alkoholkomponente in Stufe a) 1,0 bis 20,0 mmol pro 10 ml Solvens beträgt.

20. Verfahren nach den Ansprüchen 13 bis 19, **dadurch gekennzeichnet, daß** man in Stufe b) die Umsetzung mit Natriumthiosulfat in einem Lösemittelgemisch bestehend aus einem Alkohol und Wasser bei Temperaturen zwischen 50 und 100 °C durchführt.

21. Verfahren nach Anspruch 20, **dadurch gekennzeichnet, daß** man als Alkohol Methanol oder Ethanol heranzieht.

22. Verfahren nach den Ansprüchen 13 bis 21, **dadurch gekennzeichnet, daß** man in Stufe b) pro mmol Phenylalkylchlorid 1,5 bis 2,5 mmol Natriumthiosulfat verwendet.

23. Verfahren nach den Ansprüchen 13 bis 22, **dadurch gekennzeichnet, daß** man in Stufe b) die Chlorierung in Wasser, in einem Wasser/Essigsäure- oder Wasser/Dichlormethan-Gemisch bei Temperaturen zwischen 0 und +25 °C durchführt.

24. Verfahren nach den Ansprüchen 13 bis 23, **dadurch gekennzeichnet, daß** man die Umsetzung gemäß Stufe c) in einem Lösemittelgemisch bestehend aus Dichlormethan und Pyridin bei Temperaturen zwischen -60 und 0 °C vornimmt.

25. Verfahren nach den Ansprüchen 13 bis 24, **dadurch gekennzeichnet, daß** das Molverhältnis Nucleosid zu Phenylalkylsulfonylchlorid 1 : 1 bis 1 : 2 beträgt.

26. Verfahren nach den Ansprüchen 13 bis 25, **dadurch gekennzeichnet, daß** man in Stufe c) das in Pyridin gelöste Nucleosid vorlegt und eine Lösung des Phenylalkylsulfonylchlorids in Dichlormethan bei der jeweiligen Reaktionstemperatur zutropfen läßt.

27. Verfahren nach den Ansprüchen 13 bis 26, **dadurch gekennzeichnet, daß** die Konzentration des Nucleosids im Lösemittelgemisch in Stufe c) 0,1 bis 3,0 mmol pro 10 ml Solvens beträgt.

28. Verfahren nach den Ansprüchen 13 bis 27, **dadurch gekennzeichnet, daß** man die Einführung der Phosphitamid-Gruppe (Stufe d) durch Umsetzung der Nucleosid-Derivate (mit R⁵ = H) mit den entsprechenden Phosphinen in Gegenwart von 1H-Tetrazol als Aktivator in einem Lösemittelgemisch bestehend aus Dichlormethan und Acetonitril bei Temperaturen zwischen 0 und 25 °C vornimmt.

29. Verwendung eines Nucleosid-Derivats der allgemeinen Formel (I) gemäß Anspruch 1 für die lichtgesteuerte Synthese von Oligonucleotiden.

30. Verwendung gemäß Anspruch 29, **dadurch gekennzeichnet, daß** die lichtgesteuerte Oligonucleotidsynthese auf einem festen Trägermaterial erfolgt.

## Claims

1. Nucleoside derivatives having photolabile protective groups of the general formula (I) 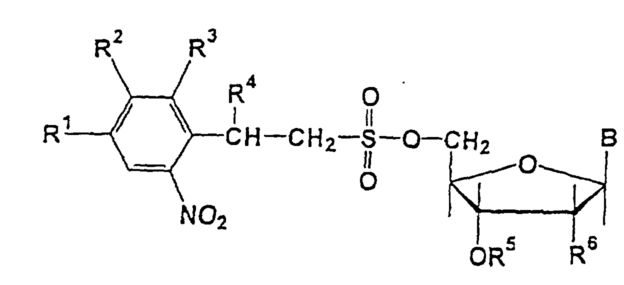 in which
R¹ = H, NO₂, CN, OCH₃, halogen or alkyl or alkoxyalkyl having 1 to 4 C atoms
R² = H, OCH₃
R³ = H, F, Cl, Br, NO₂ or an aliphatic acyl radical having 2 to 5 C atoms
R⁴ = H, halogen, OCH₃, an alkyl radical having 1 to 4 C atoms or an optionally substituted aryl radical
R⁵ = H or a conventional functional group for producing oligonucleotides
R⁶ = H, OH, halogen or XR⁸, wherein X = O or S and R⁸ represents a protective group which is conventional in nucleotide chemistry
**B = adenine**, cytosine, guanine, thymine, uracil, 2,6-diaminopurin-9-yl, hypoxanthin-9-yl, 5-methylcytosin-1-yl, 5-amino-4-imidazolcarboxylic acid amid-1-yl or 5-amino-4-imidazolcarboxylic acid amid-3-yl, wherein in the case of
B = adenine, cytosine or guanine, the primary amino function optionally has a permanent protective group.

2. Nucleoside derivatives according to claim 1, **characterised in that** in the case of R⁴≠ H, R¹ = R² = R³ = H.

3. Nucleoside derivatives according to claims 1 and 2, **characterised in that** R⁴ represents a methyl radical.

4. Nucleoside derivatives according to claims 1 to 3, **characterised in that** R⁵ represents a phosphite amide group of the formula 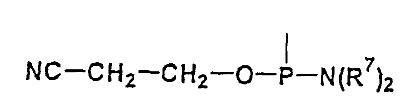 or 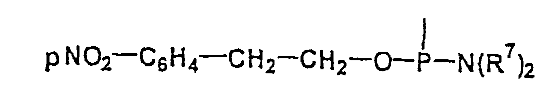 wherein the R⁷ groups are the same or different and denote linear or branched alkyl radicals having 1 to 4 C atoms.

5. Nucleoside derivatives according to claim 4, **characterised in that** R⁷ represents an ethyl or isopropyl radical.

6. Nucleoside derivatives according to claims 1 to 5, **characterised in that** R⁶ is a group XR⁸ and R⁸ in the case of X = O represents an alkyl, alkenyl, acetal or silyl ether protective group or in the case of X = S represents an alkyl protective group.

7. Nucleoside derivatives according to claim 6, **characterised in that** an O-methyl or O-ethyl radical, an O-allyl radical, an O-tetrahydropyranyl or O-methoxytetrahydropyranyl radical or an O-t-butyl dimethylsilyl radical is used as R⁶.

8. Nucleoside derivatives according to claims 1 to 7, **characterised in that** in the case of B = adenine, cytosine or guanine, phenoxyacetyl or dimethylformamidino radicals are used as permanent protective group.

9. Nucleoside derivatives according to claims 1 to 7, **characterised in that** in the case of B = adenine, benzoyl or p-nitrophenylethoxycarbonyl-(p-NPEOC) radicals are used as permanent protective group.

10. Nucleoside derivatives according to claims 1 to 7, **characterised in that** in the case of B = guanine, isobutyroyl, p-nitrophenylethyl-(p-NPE) or p-NPEOC radicals represent the permanent protective group.

11. Nucleoside derivatives according to claims 1 to 7, **characterised in that** in the case of B = cytosine, benzoyl or p-NPEOC radicals are used as permanent protective group.

12. Nucleoside derivatives according to claims 1 to 11, **characterised in that** a fluorine, chlorine or bromine atom is used as halogen atom in R¹ or R⁶.

13. Process for producing nucleoside derivatives according to claims 1 to 12 in at least three stages, **characterised in that**
a) an alcohol of the general formula (II) 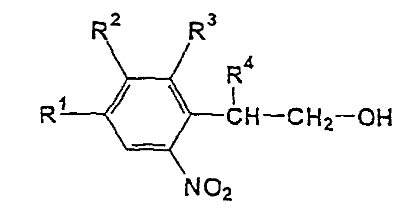 in which R¹, R², R³, R⁴ have the meaning given above, is reacted with thionyl chloride and then
b) the phenylalkyl chloride formed in stage a) of the general formula (III) 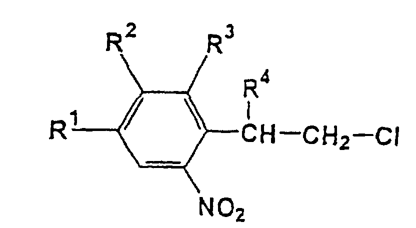 is converted initially using sodium thiosulphate and then using chlorine to form a phenylalkylsulphonyl chloride of the general formula (IV) 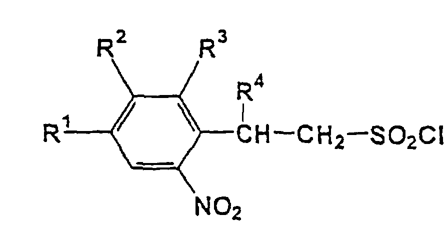 and then
c) the phenylalkylsulphonyl chloride formed in stage b) is allowed to react with nucleosides of the general formula (V) 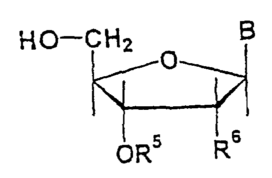 in which R⁵, R⁶ and B have the meaning given above, and optionally then
d) in the 3' position of the nucleoside derivative where R⁵ = H, the phosphite amide group 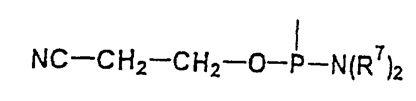 or 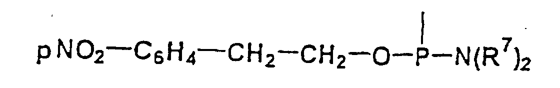 is introduced by known methods.

14. Process according to claim 13, **characterised in that** stage a) is carried out in a non-polar organic solvent at temperatures between 50 and 120°C optionally in the presence of a base.

15. Process according to claims 13 and 14, **characterised in that** in stage a) the thionyl chloride is used in two to five times excess based on the alcohol component.

16. Process according to claims 13 to 15, **characterised in that** in stage a) toluene is used as non-polar organic solvent.

17. Process according to claims 13 to 16, **characterised in that** in stage a) pyridine is used as base.

18. Process according to claim 17, **characterised in that** the pyridine is used in a quantity of 2 to 10 volume % based on the toluene used.

19. Process according to claims 13 to 18, **characterised in that** the concentration of the alcohol component in stage a) is 1.0 to 20.0 mmoles per 10 ml of solvent.

20. Process according to claims 13 to 19, **characterised in that** in stage b) the reaction with sodium thiosulphate is carried out in a solvent mixture consisting of an alcohol and water at temperatures between 50 and 100°C.

21. Process according to claim 20, **characterised in that** methanol or ethanol is used as alcohol.

22. Process according to claims 13 to 21, **characterised in that** in stage b) 1.5 to 2.5 mmoles of sodium thiosulphate are used per mmole of phenylalkyl chloride.

23. Process according to claims 13 to 22, **characterised in that** in stage b), the chlorination is carried out in water, in a water/acetic acid or water/dichloromethane mixture, at temperatures between 0 and +25°C.

24. Process according to claims 13 to 23, **characterised in that** the reaction according to stage c) is carried out in a solvent mixture consisting of dichloromethane and pyridine at temperatures between -60 and 0°C.

25. Process according to claims 13 to 24, **characterised in that** the molar ratio of nucleoside to phenylalkylsulphonyl chloride is 1:1 to 1:2.

26. Process according to claims 13 to 25, **characterised in that** in stage c) the nucleoside dissolved in pyridine is initially provided and a solution of the phenylalkylsulphonyl chloride in dichloromethane is allowed to be added dropwise at the particular reaction temperature.

27. Process according to claims 13 to 26, **characterised in that** the concentration of the nucleoside in the solvent mixture in stage c) is 0.1 to 3.0 mmoles per 10 ml of solvent.

28. Process according to claims 13 to 27, **characterised in that** the introduction of the phosphite amide group (stage d) is carried out by reacting the nucleoside derivatives (where R⁵ = H) with the corresponding phosphines in the presence of 1H-tetrazole as activator in a solvent mixture consisting of dichloromethane and acetonitrile at temperatures between 0 and 25°C.

29. Use of a nucleoside derivative of the general formula (I) according to claim 1 for the light-controlled synthesis of oligonucleotides.

30. Use according to claim 29, **characterised in that** the light-controlled oligonucleotide synthesis takes place on a solid carrier material.

## Revendications

1. Dérivés de nucléosides à groupes protecteurs photolabiles de formule générale (I) 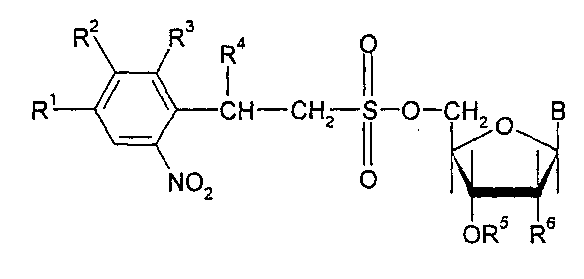 où
R¹ = H, NO₂, CN, OCH₃, halogène ou alkyle ou alcoxyalkyle de 1 à 4 atomes de carbone
R² = H, OCH₃
R³ = H, F, Cl, Br, NO₂ ou un reste acyle aliphatique de 2 à 5 atomes de carbone
R⁴ = H, halogène, OCH₃, un reste alkyle de 1 à 4 atomes de carbone ou un reste aryle éventuellement substitué
R⁵ = H ou un groupe fonctionnel courant pour la préparation d'oligonucléotides
R⁶ = H, OH, halogène ou XR⁸, où X = O ou S et R⁸ représente un groupe protecteur courant en chimie des nucléotides
B = adénine, cytosine, guanine, thymine, uracile, 2,6-diaminopurin-9-yle, hypoxanthin-9-yle, 5-méthylcytosin-1-yle, 5-amine-4-imidazolcarboxamid-1-yle ou 5-amino-4-imidazolcarboxamid-3-yle, où dans le cas où B = adénine, cytosine ou guanine, la fonction amino primaire comporte éventuellement un groupe protecteur permanent.

2. Dérivés de nucléosides selon la revendication 1 **caractérisés en ce que**, dans le cas où R⁴ ≠ H, R¹ = R² = R³ = H.

3. Dérivés de nucléosides selon les revendications 1 et 2 **caractérisés en ce que** R⁴ représente un reste méthyle.

4. Dérivés de nucléosides selon les revendications 1 à 3 **caractérisés en ce que** R⁵ représente un groupe phosphitamide de formule 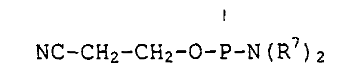 ou 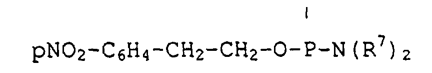 où les groupes R⁷ sont identiques ou différents et représentent des restes alkyle linéaires ou ramifiés de 1 à 4 atomes de carbone.

5. Dérivés de nucléosides selon la revendication 4 **caractérisés en ce que** R⁷ représente un reste éthyle ou isopropyle.

6. Dérivés de nucléosides selon les revendications 1 à 5 **caractérisés en ce que** R⁶ est un groupe XR⁸ et R⁸, dans le cas où X = O, représente un groupe protecteur alkyle, alcényle, acétal ou silyléther ou, dans le cas où X = S, un groupe protecteur alkyle.

7. Dérivés de nucléosides selon la revendication 6 **caractérisés en ce qu'**un reste O-méthyle ou O-éthyle, un reste O-allyle, un reste O-tétrahydropyranyle ou O-méthoxytétrahydropyranyle ou un reste O-t-butyldiméthylsilyle est utilisé comme R⁶.

8. Dérivés de nucléosides selon les revendications 1 à 7 **caractérisés en ce que**, dans le cas où B = adénine, cytosine ou guanine, on utilise des restes phénoxyacétyle ou diméthylformamidino comme groupe protecteur permanent.

9. Dérivés de nucléosides selon les revendications 1 à 7 **caractérisés en ce que**, dans le cas où B = adénine, on utilise des restes benzoyle ou p-nitrophényléthoxycarbonyle (p-NPEOC) comme groupe protecteur permanent.

10. Dérivés de nucléosides selon les revendications 1 à 7 **caractérisés en ce que**, dans le cas où B = guanine, le groupe protecteur permanent représente des restes isobutyryle, p-nitrophényléthyle (p-NPE) ou p-NPEOC.

11. Dérivés de nucléosides selon les revendications 1 à 7 **caractérisés en ce que**, dans le cas où B = cytosine, on utilise des restes benzoyle ou p-NPEOC comme groupe protecteur permanent.

12. Dérivés de nucléosides selon les revendications 1 à 11 **caractérisés en ce qu'**un atome de fluor, de chlore ou de brome est utilisé comme atome d'halogène dans R¹ ou R⁶.

13. Procédé de préparation de dérivés de nucléosides selon les revendications 1 à 12 en au moins trois étapes, **caractérisé en ce que** l'on fait réagir
a) un alcool de formule générale (II) 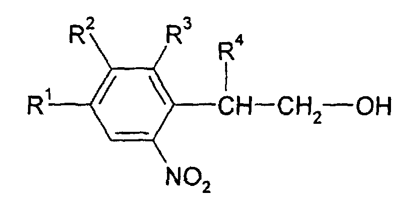 où R¹, R², R³, R⁴ possèdent la signification indiquée ci-dessus, avec le chlorure de thionyle puis
b) on convertit le chlorure de phénylalkyle de formule générale (III) 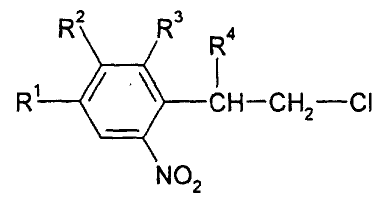 formé dans l'étape a) d'abord avec le thiosulfate de sodium puis avec le chlore en un chlorure de phénylalkylsulfonyle de formule générale (IV) 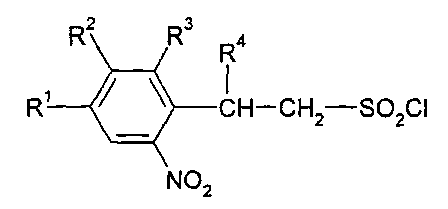 puis
c) on fait réagir le chlorure de phénylalkylsulfonyle formé dans l'étape b) avec des nucléosides de formule générale (V) 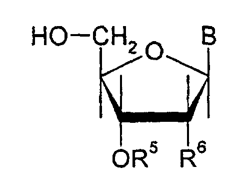 où R⁵, R⁶ et B possèdent la signification indiquée ci-dessus, puis, éventuellement
d) on introduit dans la position 3' des dérivés de nucléosides avec R⁵ = H le groupe phosphitamide 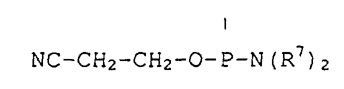 ou 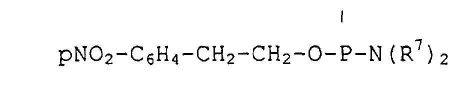 selon des méthodes connues.

14. Procédé selon la revendication 13 **caractérisé en ce que** l'on conduit l'étape a) dans un solvant organique non polaire à des températures situées entre 50 et 120°C, éventuellement en présence d'une base.

15. Procédé selon les revendications 13 et 14 **caractérisé en ce que**, dans l'étape a), on utilise le chlorure de thionyle en un excès de deux à cinq fois par rapport au composant alcool.

16. Procédé selon les revendications 13 à 15 **caractérisé en ce que**, dans l'étape a), on utilise le toluène comme solvant organique non polaire.

17. Procédé selon les revendications 13 à 16 **caractérisé en ce que**, dans l'étape a), on utilise la pyridine comme base.

18. Procédé selon la revendication 17 **caractérisé en ce que** l'on utilise la pyridine en une quantité de 2 à 10 % en volume par rapport au toluène utilisé.

19. Procédé selon les revendications 13 à 18 **caractérisé en ce que** la concentration du composant alcool dans l'étape a) est de 1,0 à 20,0 mmol pour 10 ml de solvant.

20. Procédé selon les revendications 13 à 19 **caractérisé en ce que**, dans l'étape b), on conduit la réaction avec le thiosulfate de sodium dans un mélange de solvants consistant en un alcool et en eau à des températures situées entre 50 et 100°C.

21. Procédé selon la revendication 20 **caractérisé en ce que** l'on utilise le méthanol ou l'éthanol comme alcool.

22. Procédé selon les revendications 13 à 21 **caractérisé en ce que**, dans l'étape b), on utilise 1,5 à 2,5 mmol de thiosulfate de sodium par mmol de chlorure de phénylalkyle.

23. Procédé selon les revendications 13 à 22 **caractérisé en ce que**, dans l'étape b), on conduit la chloration dans l'eau, dans un mélange eau/acide acétique ou eau/dichlorométhane à des températures situées entre 0 et +25°C.

24. Procédé selon les revendications 13 à 23 **caractérisé en ce que** l'on conduit la réaction selon l'étape c) dans un mélange de solvants consistant en dichlorométhane et en pyridine à des températures situées entre -60 et 0°C.

25. Procédé selon les revendications 13 à 24 **caractérisé en ce que** le rapport molaire nucléoside à chlorure de phénylalkylsulfonyle est de 1:1 à 1:2.

26. Procédé selon les revendications 13 à 25 **caractérisé en ce que**, dans l'étape c), on dispose au préalable le nucléoside dissous dans la pyridine et on ajoute goutte à goutte une solution du chlorure de phénylalkylsulfonyle dans le dichlorométhane à la température de réaction correspondante.

27. Procédé selon les revendications 13 à 26 **caractérisé en ce que** la concentration du nucléoside dans le mélange de solvants dans l'étape c) est de 0,1 à 3,0 mmol pour 10 ml de solvant.

28. Procédé selon les revendications 13 à 27 **caractérisé en ce que** l'on réalise l'introduction du groupe phosphitamide (étape d) par réaction des dérivés de nucléosides (avec R⁵ = H) avec les phosphines correspondantes en présence de 1H-tétrazole comme activateur dans un mélange de solvants consistant en dichlorométhane et en acétonitrile à des températures situées entre 0 et 25°C.

29. Utilisation d'un dérivé de nucléoside de formule générale (I) selon la revendication 1 pour la synthèse d'oligonucléotides commandée par la lumière.

30. Utilisation selon la revendication 29 **caractérisée en ce que** la synthèse d'oligonucléotides commandée par la lumière a lieu sur un support solide.
